# EUROPÄISCHE PATENTSCHRIFT

(11) **EP 1 204 335 B1**
(45) Veröffentlichungstag und Bekanntmachung des Hinweises auf die Patenterteilung: **02.05.2003**
(21) Anmeldenummer: 00953143.5
(22) Anmeldetag: 02.08.2000
(51) Int. Cl.: A41C 3/06, A61F 13/14

(54) **VERFAHREN ZUR VERSCHÖNERUNG DER FORM DES MENSCHLICHEN KÖRPERS MITTELS EINES KOSMETIKPFLASTERS**
METHOD FOR EMBELLISHING THE SHAPE OF A HUMAN BODY BY MEANS OF A COSMETIC PLASTER
PROCEDE D'EMBELLISSEMENT DE LA FORME DU CORPS HUMAIN AU MOYEN D'UN PANSEMENT COSMETIQUE

(30) Priorität: 03.08.1999 DE 19936395
(43) Veröffentlichungstag der Anmeldung: 15.05.2002
(73) Patentinhaber: Buntz, Antonia, 80803 München (DE)
(72) Erfinder: Buntz, Antonia, 80803 München (DE)
(74) Vertreter: Preissner, Nicolaus
(86) Internationale Anmeldenummer: EP0007491
(87) Internationale Veröffentlichungsnummer: WO01008515

(56) Entgegenhaltungen:
- FR-A- 2 388 517
- GB-A- 1 435 853
- US-A- 4 343 313
- US-A- 4 982 450
- US-A- 5 961 986

## Beschreibung

Die Erfindung betrifft ein Verfahren zum Anheben der weiblichen Brust mittels eines Pflasters, ein dazu zu verwendbares Kosmetikpflaster und die Verwendung eines Kosmetikpflasters zum Anheben der weiblichen Brust.

Zur Verschönerung des menschlichen Körpers werden zahlreiche kosmetische Verfahren und Produkte angeboten. Unter "Kosmetik" ist die Kunst der Erhaltung, Verbesserung und Wiederherstellung der Schönheit des menschlichen Körpers zu verstehen (der Große Brockhaus, 18. Auflage, 1979). Im weitesten Sinne ist darunter beispielsweise auch Unterwäsche zu verstehen, soweit diese (auch) dazu bestimmt ist, bestimmte Körperbereiche in eine optisch schöne Körperform zu bringen, wie beispielsweise Push-Up-Büstenhalter. Im engeren Sinne betrifft die Kosmetik die Verschönerung der Haut.

Aus dem deutschen Gebrauchsmuster DE 88 11 658 U1 ist beispielsweise ein Hautdress bekannt, das aus einem selbstklebenden, der Haut äußerlich angepaßten Material besteht und mit dem Falten, Narben oder Flecken auf der Haut abgedeckt werden können.

Aus der deutschen Patentanmeldung DE 43 30 506 A1 ist des weiteren ein Pflaster zur Bekämpfung vorzeitiger Faltenbildung und trockener Haut bekannt, indem Wirkstoffe von dem Pflaster beziehungsweise von einem an dem Pflaster haftenden Wirkstoffträger aus flexiblem Kunststoff an die Haut abgegeben werden. Dazu wird der zu behandelnde Hautbereich mittels zwei Fingern gespreizt, und das Pflaster wird so aufgebracht, daß die Wirkstoffe in den geglätteten und gedehnten Hautbereich eindringen können.

Die genannten Maßnahmen betreffen jedoch nur die Verschönerung der Haut des menschlichen Körpers. Eine optisch schönere Körperform ist damit nicht erzielbar. Mit solchen Maßnahmen läßt sich bestenfalls auf lange Sicht eine Verschönerung der Form des Körpers erzielen. Eine optische Verschönerung der Form von insbesondere schlaffen, großflächigen Gewebebereichen wie am Busen, Bauch, Gesäß, Oberschenkel, etc., den typischen Problemzonen, ist damit kurzfristig nicht zu erreichen.

Dagegen ist aus der WO 97/09951 ein selbstklebendes Pflaster zur äußeren medizinischen Behandlung von hypertrophen oder keloidalen Narben als Folge von Brustoperationen bekannt. Das Pflaster wird unterhalb der weiblichen Brust angebracht und weist im wesentlichen die Form eines Schiffankers auf.

Weiterhin ist aus der WO 98/34652 eine Brustbedeckung in Form eines Büstenhalters bekannt, die einen oberen, aus textilem Material bestehenden Teil und einen unteren, mit einer Klebebefestigung versehenen Teil aufweist.

Die WO 80/00121 beschreibt einen Büstenhalter, der aus einer zum Teil klebenden Folie besteht und auf der Seite beziehungsweise den Seiten der Brust haftet.

Aufgabe der vorliegenden Erfindung ist es, Körperpartien des menschlichen Körpers in eine schöne, ansprechende Form zu bringen. Eine weitergehende Aufgabe besteht in der kosmetischen Verschönerung der Haut selbst.

Diese Aufgabe wird erfindungsgemäß durch die Merkmale der nebengeordneten Verfahrens- und Produkt- und Verwendungsansprüche gelöst. In den davon abhängigen Unteransprüchen sind vorteilhafte Weiterbildungen der Erfindung angegeben.

Der Erfindung liegt das grundsätzliche Prinzip zugrunde, schlaffe oder lockere Gewebepartien mittels eines Pflasters mit straffen beziehungsweise festen Gewebepartien zu verbinden und zu den straffen Gewebepartien hinzuziehen. Indem sich dadurch die äußere Form der lockeren Gewebepartien verändert, wirkt das lockere Gewebe optisch gestrafft. Diese optische Wirkung ist am intensivsten, wenn die Straffung entgegen der Schwerkraft wirkt, wenn also das schlaffe Gewebe bei aufrechter Körperhaltung nach oben gezogen wird. Zu diesem Zweck wird ein Kosmetikpflaster auf eine über dem lockeren Gewebe liegende Hautstelle einerseits und auf eine über dem festen Gewebe liegende Hautstette andererseits geklebt, so daß es unter Zugbelastung steht, wobei die erste, über dem lockeren Gewebe liegende Befestigungsstelle oberhalb von der zweiten, über dem festen Gewebe liegenden Befestigungsstelle angeordnet ist. Vorzugsweise liegt die zweite Befestigungsstelle in unmittelbarer Nähe zum Knochen- beziehungsweise Knorpelgerüst des menschlichen Körpers.

Durch diese Maßnahmen lassen sich vor allem die Brüste aber auch Bauch, Gesäß und Oberschenkelpartien anheben, so daß ihre Form ansprechender und straffer wirkt. Auf Büstenhalter kann dann verzichtet werden. Statt dessen wird das Kosmetikpflaster in der beschriebenen Weise einfach "aufgetaped". Insbesondere die weibliche Brustwarze läßt sich mit dem Kosmetikpflaster durch geringes Anheben in eine optisch ansprechendere Position bringen und optisch hervorheben. Durch Büstenhalter verursachen Hauteinschnürungen, die durch die Kleidung hindurch häufig zu sehen sind, treten nicht mehr auf, so daß die optische Wirkung der Körperform noch gefälliger wird. Das Kosmetikpflaster kann aber auch zusätzlich zum Büstenhalter verwendet werden, um beispielsweise beim Sporttreiben oder bei anderen körperlichen Aktivitäten unterstützend zu wirken.

Das Kosmetikpflaster ist sowohl für kurzfristigen als auch für langfristigen Gebrauch geeignet und wird vorzugsweise zwischen einigen Stunden und drei Tagen getragen.

Bevorzugte Eigenschaften des Kosmetikpflasters sind Wasserbeständigkeit, Luftdurchlässigkeit, Wasserdampfdurchlässigkeit, extrem geringe Dicke, Transparenz oder zumindest Hautfarbe, Elastizität, Klebefähigkeit im wesentlichen über die gesamte Pflasterfläche (vorzugsweise zumindest zwischen den Befestigungsstellen) und Dotierung mit Aromastoffen.

Die Wasserbeständigkeit gestattet es dem Träger, sich bei mehrtägigem Tragen mit angelegtem Kosmetikpflaster zu duschen oder zu baden.

Die Luftdurchlässigkeit und Wasserdampfdurchlässigkeit ist vorteilhaft, um die Funktion der Respiration der Haut aufrechtzuerhalten. Das Trägermaterial des Pflasters weist zu diesem Zweck feine Poren auf, und auch die Klebefläche ist diskontinuierlich, beispielsweise netzartig oder mit Poren, ausgebildet.

Die geringe Dicke des Kosmetikpflasters ist für den optischen Eindruck vorteilhaft. Das Pflaster sollte so dünn sein, daß es bei eng anliegender dünner Kleidung, beispielsweise T-Shirts, Bodies, etc. nicht durch die Kleidung hindurch erkennbar ist.

Vorteilhafterweise ist das Kosmetikpflaster transparent, so daß es bei durchscheinender Kleidung nicht erkennbar ist und auch nicht sofort auffällt, wenn durch Verrutschen des Kleidungsstücks der Blick direkt auf das Kosmetikpflaster möglich ist. Auch hautfarbene Kosmetikpflaster sind geeignet, wenn auch auf Grund der Farbnuancen der menschlichen Haut nicht so geeignet wie transparentes Material.

Elastizität des Kosmetikpflasters ist ebenfalls sehr vorteilhaft. Dadurch wird verhindert, daß die Gewebepartie unter dem Kosmetikpflaster gegenüber den benachbarten Gewebepartien absolut fixiert ist. Eine solche absolute Fixierung könnte bei rascher Körperbewegung erkennbar werden, wenn sich die benachbarten Gewebepartien in auffälliger Weise relativ zu der fixierten Gewebepartie verlagern würden. Durch die Elastizität des Kosmetikpflastermaterials wirkt das Kosmetikpflaster wie ein Expander zwischen den beiden Befestigungspunkten, wobei in jedem Zeitpunkt die Funktion des Kosmetikpflasters, die darin besteht, die lockeren Gewebepartien anzuheben, gewährleistet ist. Ein unelastisches Kosmetikpflaster führt außerdem je nach der vom Körper eingenommenen Position zu unangenehmer und unschöner Faltenbildung.

Die Klebefähigkeit des Kosmetikpflasters im wesentlichen über die gesamte Pflasterfläche ist insbesondere bei solchen Anwendungsfällen vorteilhaft, wo zwischen den beiden Befestigungsstellen eine konkave Krümmung der Körperoberfläche vorliegt. Das Kosmetikpflaster würde in diesen Fällen zwischen den Befestigungspunkten von der Hautoberfläche abheben. Die durchgehende Klebefähigkeit ist daher bei der Verwendung des Kosmetikpflasters zur Anhebung der weiblichen Brust besonders vorteilhaft, aber auch für andere Anwendungsbereiche sinnvoll.

Als geeignetes Material für das Kosmetikpflaster hat sich beispielsweise Tegaderm™ der Firma 3M erwiesen.

Durch Dotierung des Kosmetikpflasters mit Aromastoffen kann das Kosmetikpflaster zusätzliche Funktionen, beispielsweise nach Art eines Deodorants, ausüben.

Es ist weiterhin vorteilhaft, wenn dem Kosmetikpflaster Wirkstoffe zugesetzt sind, die nach und nach an die Haut abgegeben werden. Die Wirkstoffe können beispielsweise zur Funktionsunterstützung der Haut, gegen Zellulitis oder zu anderen Zwecken bestimmt sein. Zur Bekämpfung von Cellulistis können beispielsweise Kampferpräparate oder Eukalyptuspräparate dem Kosmetikpflaster zugesetzt sein. Als weitere Wirkstoffe kommen beispielsweise Vitamine, Mineralien, Kräuteressenzen etc. in Betracht.

An Hand der nachfolgenden Figuren werden einige vorteilhafte Ausgestaltungen des erfindungsgemäßen Kosmetikpflasters beispielhaft beschrieben:
- Fig. 1: zeigt eine menschlichen Körper mit Kosmetikpflastem an verschiedenen Körperstellen;
- Fig. 2a und 2b: zeigen erfindungsgemäße Körperpflaster zum Anheben der Brüste;
- Fig. 3: zeigt ein Kosmetikpflaster zum optischen Straffen der Bauchpartie des menschlichen Körpers; und
- Fig. 4a und 4b: zeigen Kosmetikpflaster zum Anheben der Gesäßhälften des menschlichen Körpers.

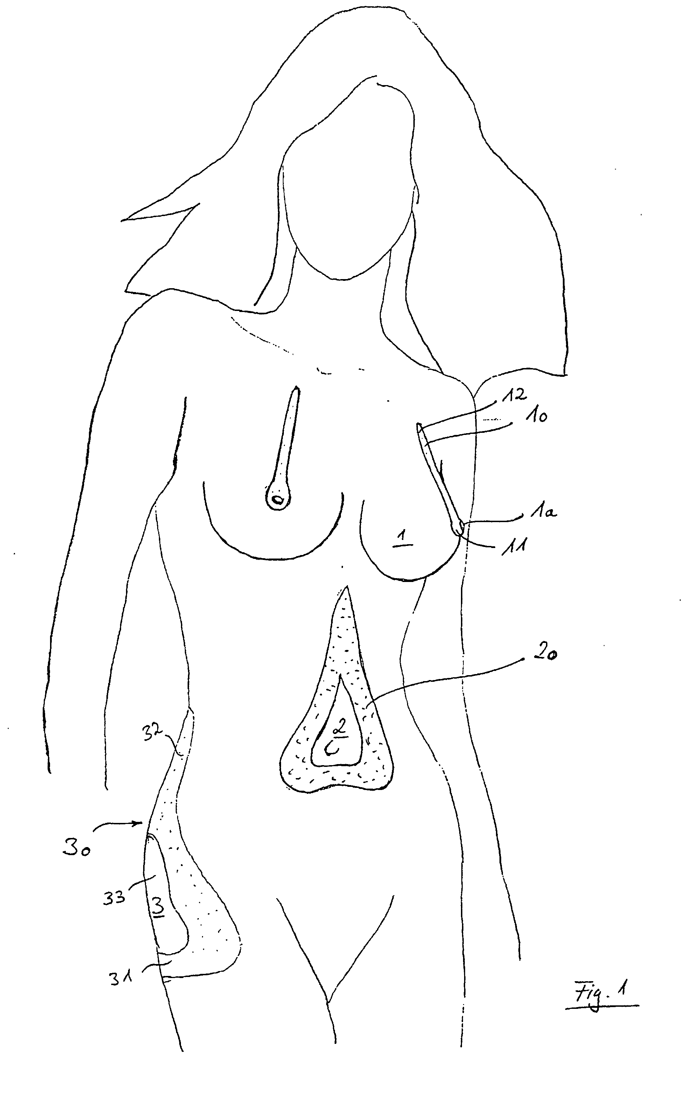

In Fig. 1 ist ein weiblicher Körper dargestellt, auf den drei verschiedene Kosmetikpflaster durch einfaches "Tapen" aufgeklebt sind. Ein Kosmetikpflaster 10 ist an jeder Brust 1 des menschlichen Körpers befestigt. Dabei umschließt ein erster Befestigungsbereich 11 des Kosmetikpflasters 10 die Brustwarze 1a. Der zweite Befestigungsbereich 12 des Kosmetikpflasters 10 ist in der Nähe des Schlüsselbeins des menschlichen Körpers fixiert, da diese Hautpartie nur eine relativ geringe Relativbewegung zu dem darunterliegenden Knochengerüst des menschlichen Körpers erfährt. Um ein Abheben des Kosmetikpflasters 10 von der Körperoberfläche zwischen den ersten und zweiten Befestigungsbereichen 11, 12 zu vermeiden, ist das Kosmetikpflaster 10 durchgehend auf die Haut aufgeklebt.

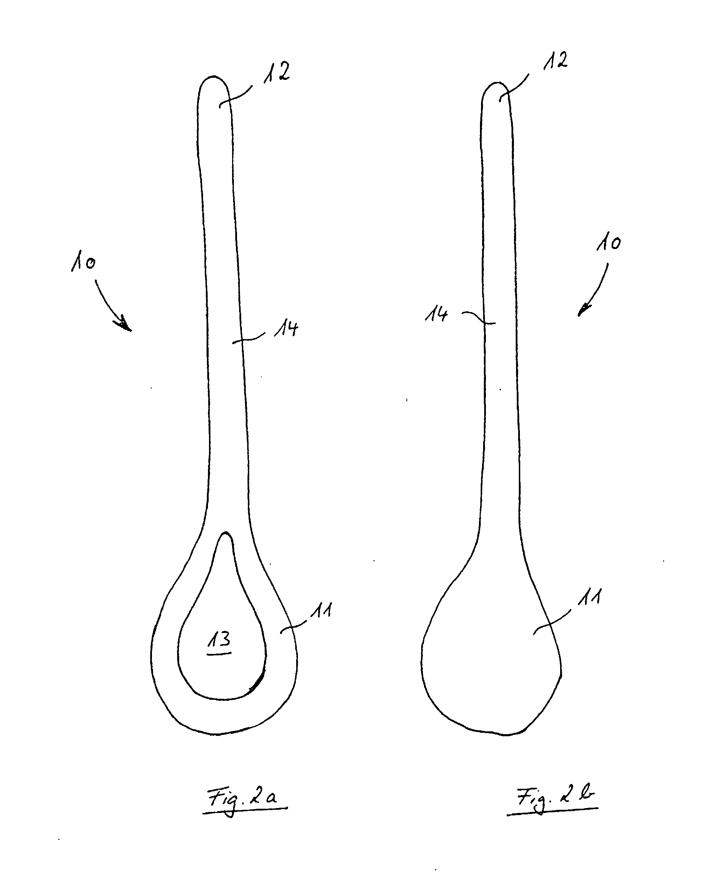

In Fig. 2a und 2b sind zwei Kosmetikpflaster 10 zur Anhebung der Brüste und Herausstellen der weiblichen Brustwarze separat dargestellt. Im Gegensatz zu normalen Pflastern ist es vorteilhaft, wenn der erste Befestigungsbereich 11, der über dem schlaffen beziehungsweise lockeren Gewebe auf der Haut fixiert wird, relativ breit ausgebildet ist. Das Pflaster verjüngt sich dann zu dem zweiten Befestigungsbereich 12 hin. Sehr vereinfacht ausgedrückt kann man von einer Dreiecksform sprechen. Der zweite Befestigungsbereich muß aber nicht notwendigerweise die schmalste Stelle des Pflasters bilden. Allerdings ist der zweite Befestigungsbereich in aller Regel schmaler als der erste Befestigungsbereich des Pflasters.

Diese grundsätzliche Struktur besitzen auch die anderen Kosmetikpflaster. Zur Durchführung des erfindungsgemäßen Verfahrens ist diese Struktur allerdings nicht zwingend notwendig, sondern es können vielmehr auch herkömmliche Pflasterformen verwendet werden.

Das Kosmetikpflaster 10 in Fig. 2a weist eine Aussparung 13 mit einer Breite von etwa 25 mm auf, durch die hindurch die Brustwarze 1a (Fig. 1) ragt. Dadurch kommt die Brustwarze 1a wesentlich besser zur Geltung, wodurch insbesondere die erotische Ausstrahlung des Körpers verstärkt wird. Das Kosmetikpflaster 10 hat in diesem Fall eine flaschenöffnerförmige Gestalt. Die Breite der durch die Aussparung 13 gebildeten Schlaufe liegt etwa bei 7 mm, während der zum zweiten Befestigungsbereich 12 führende Steg 14 etwa 10 mm breit ist. Die Gesamtlänge des Kosmetikpflasters 10 beträgt etwa 160 mm.

Die Aussparung 13 kann aber auch entfallen (Fig. 2b), so daß das Kosmetikpflaster 10 zur Anhebung der Brüste eher löffelförmig erscheint. Diese Ausführungsform ist insbesondere für Frauen bestimmt, die das optische Herausstellen der Brustwarze als unangenehm empfinden.

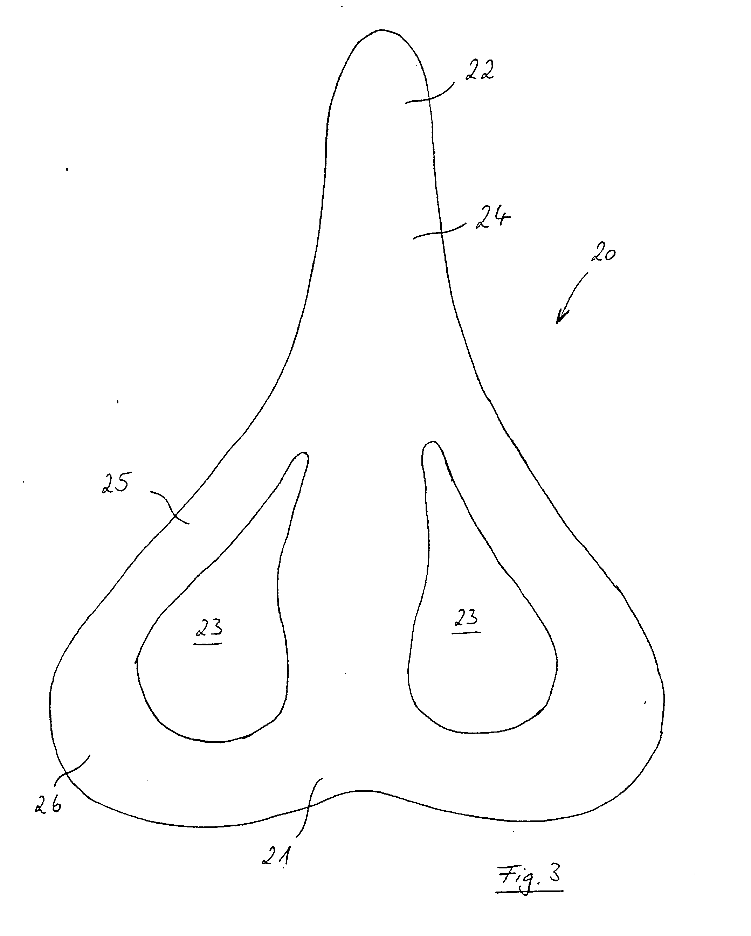

In Fig. 3 ist ein Kosmetikpflaster 20 zum Anheben der Bauchpartie 2 dargestellt. Es ist aber genauso für den Oberschenkel 3 und bedingt auch zum Anheben der Gesäßpartien geeignet. Auch hier ist der erste Befestigungsbereich 21 des Kosmetikpflasters 20 breit ausgebildet, um die untere Bauchpartie erfassen zu können, läuft dann nach oben schmal zu und endet in dem zweiten Befestigungsbereich 22. Eine Aussparung 23 ist ebenfalls vorgesehen. Die Aussparung hat insbesondere den Zweck, das Tragen des Kosmetikpflasters angenehm zu gestalten, da der Tragekomfort mit zunehmender Pflasterfläche abnimmt. Während der erste Befestigungsbereich 21 des Kosmetikpflasters 20 unterhalb des Bauchnabels der tragenden Person befestigt wird, wird der zweite Befestigungsbereich 22 in der Nähe des Solarplexus befestigt. Die bevorzugte Ausführungsform des Kosmetikpflasters 20 zum Anheben der Bauchpartie ist in etwa tropfenförmig, gegebenenfalls mit einer Delle im unteren, ersten Befestigungsbereich 21, wie in Fig. 3 dargestellt.

Das Kosmetikpflaster 20 ist etwa 125 mm lang. Der Steg 24 ist etwa 30 mm breit und die durch die Aussparungen 23 gebildeten Schlaufen haben im oberen Schlaufenbereich 25 eine Breite von etwa 20 mm und im unteren Schlaufenbereich 26 eine Breite von etwa 25 mm.

Das Kosmetikpflaster 30 zum Straffen der Außenseite des Oberschenkels 3 ist prinzipiell aufgebaut wie das Kosmetikpflaster 20 zum Anheben der Bauchpartie und ist deshalb nicht gesondert dargestellt. Wie in Fig. 1 zu sehen ist, ist der zweite Befestigungsbereich 32 in der Nähe des Hüftknochens an der Taille etwa in Höhe des Bauchnabels fixiert.

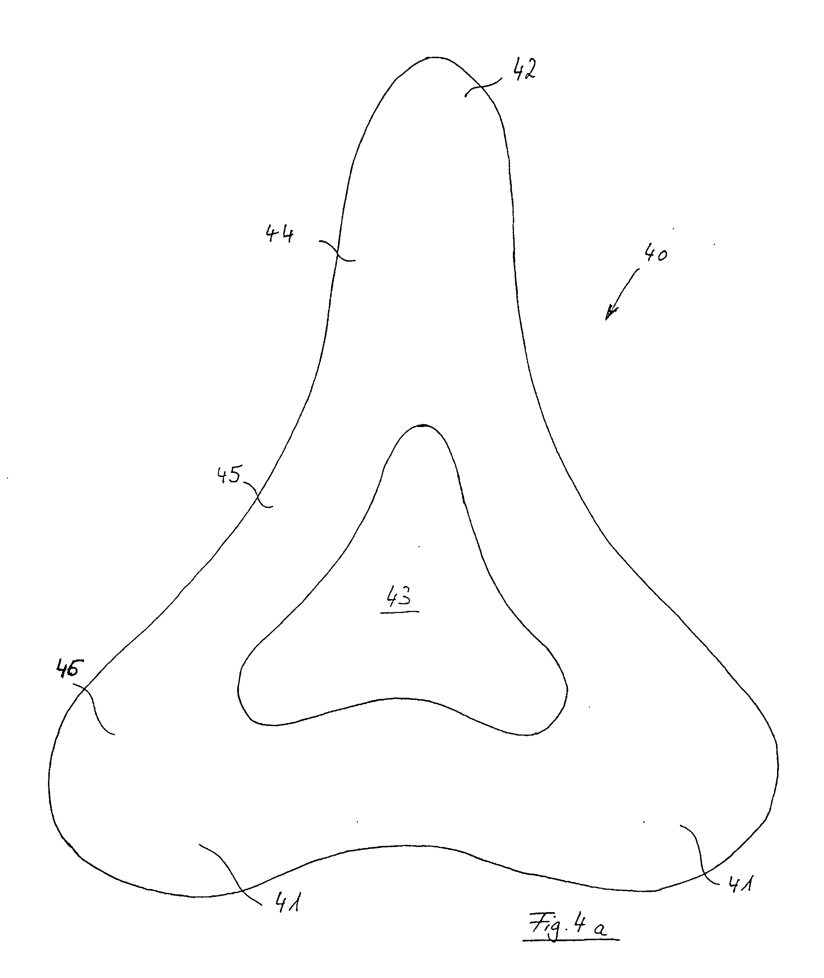

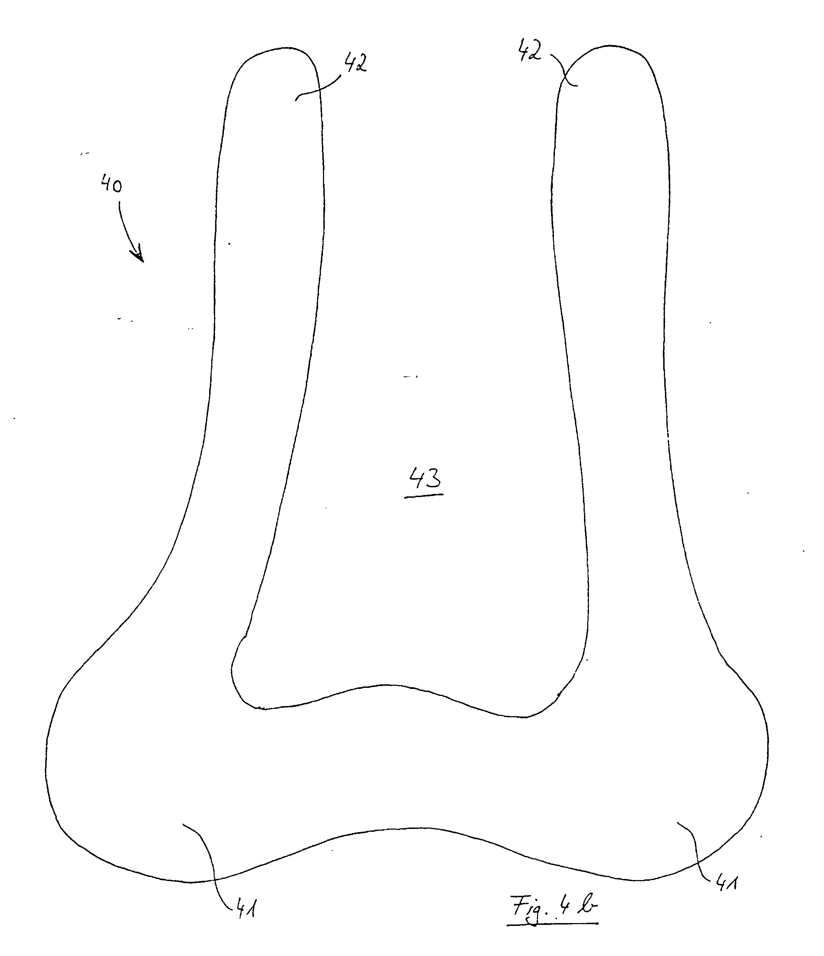

In Fig. 4a und 4b sind zwei Kosmetikpflaster zum Anheben insbesondere der Gesäßpartien dargestellt. Sie sind aber ebenso zur Verwendung am Oberschenkel und Bauch geeignet. Der erste Befestigungsbereich 41 wird auf den Gesäßpartien fixiert, während der zweite Befestigungsbereich 42 je nach Ausführungsform entweder an der Wirbelsäule in Höhe des Steißbeins (Fig. 4a) oder links und rechts neben der Wirbelsäule an den Leistenpunkten (Fig. 4b) befestigt wird. Diese Ausführungsformen können daher als herzförmig oder W-förmig bezeichnet werden. Die herzförmige Ausführungsform nach Fig. 4a ist im Längsmittelbereich etwa 200 mm lang und an der breitesten Stelle 180 mm breit. Die Breite des Stegs 44 beträgt 45 mm, während die Breite der von der Aussparung 43 gebildeten Schlaufe im oberen Schlaufenbereich 45 etwa 30 mm und im unteren Schlaufenbereich 46 etwa 55 mm beträgt.

## Patentansprüche

1. Verfahren zum Anheben der weiblichen Brust mittels eines Pflasters (10; 20; 30; 40), wobei das Pflaster einerseits an mindestens einer ersten Hautstelle des Körpers über lockerem Gewebe der weiblichen Brust und andererseits an mindestens einer zweiten Hautstelle des Körpers über festem Gewebe derart befestigt wird, daß das lockere Gewebe bei aufrechter Körperhaltung entgegen der Schwerkraft in Richtung zum festen Gewebe gezogen wird, **dadurch gekennzeichnet, daß** die erste Befestigungsstelle im Bereich der Brustwarze (1a) liegt.

2. Verfahren nach Anspruch 1, wobei die zweite Befestigungsstelle in unmittelbarer Nähe zum Knochen- oder Knorpelgerüst des menschlichen Körpers liegt.

3. Verfahren nach Anspruch 1 oder 2, wobei ein Pflaster verwendet wird, das einen bei aufrechter Körperhaltung unteren, breiten, nach oben hin schmaler werdenden, ersten Befestigungsbereich (11) zur Befestigung über dem lockeren Gewebe und einen oberen, zweiten Befestigungsbereich (12) zur Befestigung über dem festen Gewebe aufweist.

4. Verfahren nach einem der Ansprüche 1 bis 3, wobei die zweite Befestigungsstelle in der Nähe des Schlüsselbeins des menschlichen Körpers liegt.

5. Verfahren nach einem der Ansprüche 1 bis 4, wobei sich die erste Befestigungsstelle um die Brustwarzen (1a) herum erstreckt.

6. Verfahren nach einem der Ansprüche 1 bis 4, wobei die erste Befestigungsstelle die Brustwarze (1a) des menschlichen Körpers mit umfaßt.

7. Verfahren nach einem der Ansprüche 1 bis 5, wobei ein Pflaster verwendet wird, bei dem in dem unteren, breiten Befestigungsbereich eine Aussparung (13) vorgesehen ist.

8. Verfahren nach einem der Ansprüche 1 bis 7, wobei ein Pflaster verwendet wird, das zwischen der ersten Befestigungsstelle und der zweiten Befestigungsstelle durchgehend klebend ist.

9. Verfahren nach einem der Ansprüche 1 bis 8, wobei ein Pflaster verwendet wird, das so dünn ist, daß es unter eng anliegender Kleidung nicht erkennbar ist.

10. Kosmetikpflaster (10) zum Anheben der weiblichen Brust umfassend mindestens einen ersten Befestigungsbereich (11) zur Fixierung an einer ersten Stelle eines menschlichen Körpers über lockerem Gewebe der weiblichen Brust und mindestens einen zweiten Befestigungsbereich (12) zur Fixierung an einer zweiten Stelle des menschlichen Körpers über festem Gewebe, die miteinander verbunden sind, wobei sich der mindestens eine erste Befestigungsbereich in Richtung zu mindestens einen zweiten Befestigungsbereich verjüngt, **dadurch gekennzeichnet, daß** die Form des Kosmetikpflasters in Draufsicht in etwa der Form eines Löffels entspricht.

11. Kosmetikpflaster nach Anspruch 10, das zwischen mindestens einem ersten Befestigungsbereich und mindestens einem zweiten Befestigungsbereich durchgehend klebend ist.

12. Kosmetikpflaster nach Anspruch 10 oder 11, das transparent und/oder wasserbeständig und/oder luftdurchlässig und/oder wasserdampfdurchlässig ist.

13. Kosmetikpflaster nach einem der Ansprüche 10 bis 12, das so dünn ist, daß es unter eng anliegender Kleidung, insbesondere T-Shirt, Body, nicht erkennbar ist.

14. Kosmetikpflaster nach einem der Ansprüche 10 bis 13, das Wirkstoffe und/oder Aromastoffe enthält.

15. Kosmetikpflaster nach einem der Ansprüche 10 bis 14, wobei eine Aussparung (13) im ersten Befestigungsbereich (11) vorgesehen ist.

16. Kosmetikpflaster nach Anspruch 15, wobei die Aussparung (13) der löffelähnlichen Form des Kosmetikpflasters eine flaschenöffnerähnliche Form verleiht.

17. Verwendung eines Kosmetikpflasters nach einem der Ansprüche 10 bis 16 zum Anheben der weiblichen Brust entgegen der Schwerkraft.

## Claims

1. A method of lifting the female breast by means of a tape (10;20;330;40) attached at one end to at least one first skin site of the body over sagging tissue of the female breast and at the other end to at least one second skin site of the body over firm tissue such that said sagging tissue in the upright posture of the body is drawn against the force of gravity in the direction of said firm tissue, **characterized in that** said first attachment site is in the region of the nipple (1a).

2. The method as set forth in claim 1 wherein said second attachment site is in the direct vicinity of a skeletal component of the human body.

3. The method as set forth in claim 1 or 2 wherein a tape is used comprising in the upright posture of the body a lower, wide, upwards more narrow first attachment portion (11) for attachment over sagging tissue and an upper second attachment portion (12) for attachment over firm tissue.

4. The method as set forth in any of the claims 1 to 3 wherein said second attachment site is in the vicinity of the collar bone of the human body.

5. The method as set forth in any of the claims 1 to 4 wherein said first attachment site encircles the nipples (1a).

6. The method as set forth in any of the claims 1 to 4 wherein said first attachment site includes the nipple (1a) of the human body.

7. The method as set forth in any of the claims 1 to 5 wherein a tape is used in which an opening (13) is provided in the lower, wide attachment portion.

8. The method as set forth in any of the claims 1 to 7 wherein a tape is used which is sticky full-length between said first attachment site and said second attachment site.

9. The method as set forth in any of the claims 1 to 8 wherein a tape is used which is so thin that it is not evident under a close-fitting garment.

10. Cosmetic tape (10) for lifting the female breast comprising at least one first attachment portion (11) for attachment to a first site of the human body over sagging tissue of the female breast and at least one second attachment portion (12) for attachment to a second site of the human body over firm tissue, connected to each other, said at least one first attachment portion being tapered in the direction of said at least one second attachment portion **characterized by the** shape of the cosmetic tape as viewed from above being roughly that of a spoon.

11. The cosmetic tape as set forth in claim 10 which is sticky full-length between at least one first attachment portion and at least one second attachment portion.

12. The cosmetic tape as set forth in claim 10 or 11 which is transparent and/or waterproof and/or permeable to air and/or water vapor.

13. The cosmetic tape as set forth in any of the claims 10 to 12 which is so thin that it is not evident under a close-fitting garment, more particularly T-shirt, body.

14. The cosmetic tape as set forth in any of the claims 10 to 13 which contains active agents and/or aromatic substances.

15. The cosmetic tape as set forth in any of the claims 10 to 14 wherein an opening (13) is provided in said first attachment portion (11).

16. The cosmetic tape as set forth in claim 15 wherein said opening (13) renders said spoon-like shape of said cosmetic tape into the shape of a bottle-opener.

17. Use of a cosmetic tape as set forth in any of the claims 10 to 16 for lifting the female breast against the force of gravity.

## Revendications

1. Procédé pour relever la poitrine féminine au moyen d'un pansement (10 ; 20 ; 30 ; 40), le pansement étant fixé d'une part sur au moins un premier emplacement de la peau du corps par-dessus des tissus lâches de la poitrine féminine et d'autre part sur au moins un deuxième emplacement de la peau du corps par-dessus des tissus fermes, de telle sorte que lors d'une attitude corporelle redressée, les tissus lâches sont tirés en sens opposé à la gravité en direction des tissus fermes, **caractérisé en ce que** le premier emplacement de fixation se trouve dans la zone du mamelon (1a).

2. Procédé selon la revendication 1, dans lequel le deuxième emplacement de fixation est à proximité immédiate de l'ossature ou de la structure cartilagineuse du corps humain.

3. Procédé selon l'une ou l'autre des revendications 1 et 2, dans lequel on utilise un pansement qui comprend une première zone de fixation (11) large inférieure, allant en se rétrécissant vers le haut, lors d'une attitude corporelle redressée, destinée à la fixation par-dessus les tissus lâches, et une deuxième zone de fixation supérieure (12) destinée à la fixation par-dessus les tissus fermes.

4. Procédé selon l'une des revendications 1 à 3, dans lequel le deuxième emplacement de fixation se trouve à proximité de la clavicule du corps humain.

5. Procédé selon l'une des revendications 1 à 4, dans lequel le premier emplacement de fixation s'étend autour des mamelons (1a).

6. Procédé selon l'une des revendications 1 à 4, dans lequel le premier emplacement de fixation englobe également le mamelon (1a) du corps humain.

7. Procédé selon l'une des revendications 1 à 5, dans lequel on utilise un pansement dans lequel une échancrure (13) est prévue dans la zone de fixation inférieure large.

8. Procédé selon l'une des revendications 1 à 7, dans lequel on utilise un pansement qui est adhésif en continu entre le premier emplacement de fixation et le deuxième emplacement de fixation.

9. Procédé selon l'une des revendications 1 à 8, dans lequel on utilise un pansement qui est si mince qu'il n'est pas discernable sous un vêtement collant.

10. Pansement cosmétique (10) pour relever la poitrine féminine, comportant au moins une première zone de fixation (11) pour la fixation à un premier emplacement du corps humain par-dessus des tissus lâches de la poitrine féminine et au moins une deuxième zone de fixation (12) pour la fixation à un deuxième emplacement du corps humain par-dessus des tissus fermes, qui sont reliées l'une à l'autre, ladite au moins première zone de fixation allant en se rétrécissant en direction de ladite au moins une deuxième zone de fixation, **caractérisé en ce que** la forme du pansement cosmétique correspond en vue de dessus approximativement à la forme d'une cuillère.

11. Pansement cosmétique selon la revendication 10, qui est adhésif en continu entre au moins une première zone de fixation et au moins une deuxième zone de fixation.

12. Pansement cosmétique selon l'une ou l'autre des revendications 10 et 11, qui est transparent et/ou résistant à l'eau et/ou perméable à l'air et/ou perméable à la vapeur d'eau.

13. Pansement cosmétique selon l'une des revendications 10 à 12, qui est si mince qu'il n'est pas discernable sous un vêtement collant, en particulier un T-shirt, ou un justaucorps dit "body".

14. Pansement cosmétique selon l'une des revendications 10 à 13, qui contient des substances actives et/ou des substances aromatiques.

15. Pansement cosmétique selon l'une des revendications 10 à 14, dans lequel une échancrure (13) est prévue dans la première zone de fixation (11).

16. Pansement cosmétique selon la revendication 15, dans lequel l'échancrure (13) confère une forme similaire à un décapsuleur à la forme en cuillère du pansement cosmétique.

17. Utilisation d'un pansement cosmétique selon l'une des revendications 10 à 16 pour relever la poitrine féminine en sens opposé à la gravité.
